# EUROPEAN PATENT SPECIFICATION

(11) **EP 0 831 826 B1**
(45) Date of publication and mention of the grant of the patent: **16.10.2002**
(21) Application number: 96921929.4
(22) Date of filing: 04.06.1996
(51) Int. Cl.: A61K 31/47, A61K 47/14

(54) **PHARMACEUTICAL COMPOSITION COMPRISING A PROTEINASE INHIBITOR AND A MONOGLYCERIDE**
PHARMAZEUTISCHE ZUSAMMENSETZUNG ENTHALTEND EINEN INHIBITOR VON PROTEINASE UND EINEN MONOGLYCERID
COMPOSITIONS PHARMACEUTIQUES COMPRENANT UN INHIBITEUR DE PROTEINASES ET UN MONOGLYCERIDE

(30) Priority: 06.06.1995 US 468493; 07.05.1996 US 616233
(43) Date of publication of application: 01.04.1998
(73) Proprietor: F. HOFFMANN-LA ROCHE AG, 4070 Basel (CH)
(72) Inventor: BAILEY, Carole, Anne, Warren, NJ 07059 (US); FERDINANDO, Josephine, Christine, Chippenham Wiltshire SN15 3XW (GB); SHAH, Navnit, Clifton, NJ 07012 (US)
(74) Representative: Rauber, Beat
(86) International application number: EP9602431
(87) International publication number: WO96039142

(56) References cited:
- WO-A-95/07696
- US-A- 5 196 438

## Description

It is well known in the art that a wide range of diseases are caused by retroviruses.

As far as is known at the present, AIDS is a disease of the immune system caused by the retrovirus HIV (Human Immunodeficiency Virus). According to estimates by the World Health Organization, the disease, which affects about 10 million people, is continuing to spread and in virtually all cases results in the death of the patient.

Retroviruses HIV-1 and HIV-2 have been identified as a cause of the disease and they have been characterized by molecular biology.

Retroviral protease is a proteolytic enzyme that, owing to an aspartate residue in the active center, is regarded as an aspartate protease and participates in the maturation of new infectious virions in infected cells in the reproductive cycle of a number of retroviruses.

For example, HIV-1 and HIV-2 each have in their genome a region that codes for a "gag-protease". That "gag-protease" is responsible for the correct proteolytic cleavage of the precursor proteins that are produced from the genome regions coding for the "Group Specific Antigens" (gag). During the cleavage, the structural proteins of the virus core are liberated. The "gag-protease" itself is a component of a precursor protein encoded by the pol-genome region of HIV-1 and HIV-2, which protein also contains the regions for the "reverse transcriptase" and the "integrase" and is thought to be cleaved by autoproteolysis.

The "gag-protease" cleaves the major core protein p24 of HIV-1 and HIV-2 preferentially N-terminally of proline residues, for example in the divalent residues Phe-Pro, Leu-Pro, or Tyr-Pro. It is a protease having a catalytically active aspartate residue in the active center, a so-called aspartate protease.

As used herein, proteinase inhibitor refers to those compounds which inhibit aspartate proteases of viral origin and which are useful in the prophylaxis or treatment of viral infections caused by retroviruses, such as HIV, in mammals, both human and non-human. Details of the design of such proteinase inhibitors can be found, for example, in Roberts, N.A., et al., Science, 248, 358 (20 April 1990); Overton, H.A., et al., Virology, 179, 508 (1990); Tucker, T.J., et al., J. Med. Chem., 35, 2525 (1992); and Phylip, L.H., et al., FEBS Letters, 314, 449 (1992).

Because of the hydrophobic and/or lipophilic character of proteinase inhibitors, pharmaceutical formulations thereof with conventional solid or liquid pharmaceutical excipients tend to have disadvantages. For example the proteinase inhibitor may not be satisfactorily absorbed. Among the inherent factors known to affect absorption are the method of manufacture or method of compounding; the particle size and crystal form or polymorph of the drug substance; and the diluents and excipients used in formulating the dosage form, including carriers, fillers, binders, disintegrating agents, lubricants, coatings, solvents, suspending agents, and dyes.

A requirement for therapeutic effectiveness *in vivo* is the achievement of good bioavailability, for example good absorptive capacity and/or a high blood level, also in the case of enteral, such as oral, administration, in order to obtain sufficiently high concentrations in the infected cells and/or good distribution within a host in need of treatment.

An additional requirement is that the unit dosage form have good stability or shelf life so that it can be stored conveniently (e.g., no refrigeration, i.e., at room temperature (about 20 °C) for a long period of time (e.g., about two years).

While there are many known proposals to alleviate or overcome problems of this type, it has been found that many of these proposals are inadequate in the area of the proteinase inhibitors. It has, however, surprisingly been found that certain classes of glycerides used as carrier components of formulation do assist in alleviating these inadequacies. In particular, they enable achievement of better absorption and thus enhanced bioavailability and have good stability or shelf life over a long period of time.

The present invention accordingly provides a pharmaceutical composition, preferably in unit dose, comprising (a) a therapeutically effective amount of a proteinase inhibitor, selected from N-tert.-butyl-decahydro-2-[2(R)-hydroxy-4-phenyl-3(S)-[[N-(2-quinolylcarbonyl)-L-asparaginyl]amino]butyl]-(4aS,8aS)-isoquinoline-3(S)-carboxamide its pharmaceutically acceptable salts or esters including their salts or of N-tert.-butyl-decahydro-2-[2(R)-hydroxy-4-phenyl-3(S-[[N-benzyloxycarbonyl)-L-asparaginyl]-amino]butyl]-(4aS,8aS)-isoquinoline-3(S)-carboxamide, its pharmaceutically salts or esters including their salts; and (b) a carrier containing a monoglyceride of C₈-C₁₀ medium chain fatty acids. The amount of the monoglyceride in the pharmaceutically acceptable carrier of component (b) is at least sufficient to dissolve the proteinase inhibitor.

Preferably, the ratio of monoglyceride of component (b) to component (a) is at least 1.5, more preferably at least 2.0, further preferably at least from 2.5 to 3.5, and most preferably at least 3. Preferably, component (b) has an acid value of 2.5, more preferably less than or equal to 0.5, more preferably less than or equal to 0.26, and further preferably less than or equal to 0.1 and even more preferably less than or equal to 0.04.

The medium chain fatty acids can also be partially ethoxylated with polyethylene glycol of molecular weight of from 300 to 500, which is equivalent to six to eight moles of ethylene oxide.

Mixtures of the monoglyceride of C₈-C₁₀ medium chain fatty acids and the partially ethoxylated medium chain fatty acids can also be contemplated.

The pharmaceutically acceptable carrier can further contain polyvinylpyrrolidone.

As contemplated herein, the pharmaceutically acceptable carrier comprises at least one mono-glyceride of a medium chain fatty acid and mixtures thereof. Additionally, the pharmaceutically acceptable carrier can also contain di- and triglycerides of medium chain fatty acids and mixtures thereof. The mono-, di-, and tri-glycerides also can be partially ethoxylated. More preferably, the medium chain fatty acid glycerides are selected from the group consisting of a monoglyceride of C₈ to C₁₀ medium chain fatty acids and polyethylene glycol C₈-C₁₀ medium chain fatty acid glycerides. By medium chain fatty acids, it is meant fatty acids having from eight (8) to ten (10) carbon atoms. Examples of medium chain fatty acids are caprylic (eight carbon atoms) and capric (ten carbon atoms).

Glycerides have the formula: 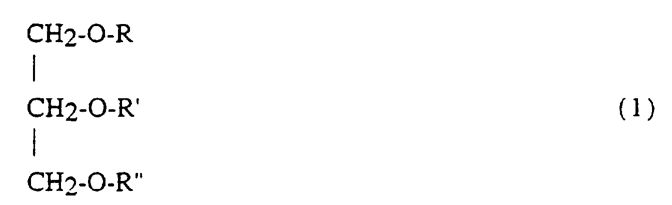 where R, R', and R" are either H or -C(O)-(CH₂)ₘ-CH₃ where m is an integer from 4 to 10 inclusive with at least one of R, R', and R" being -C(O)-(CH₂)ₘ-CH₃. In the instance where only one of R, R', and R" is -C(O)-(CH₂)ₘ-CH₃, formula (1) represents a monoglyceride. In the instance where any two of R, R', and R" are -C(O)-(CH₂)ₘ-CH₃, formula (1) represents a diglyceride. In the instance where all of R, R', and R" are -C(O)-(CH₂)ₘ-CH₃, formula (1) represents a triglyceride.

A preferable glyceride is a mixture of mono- and diglyceride of saturated C₈ to C₁₀ (e.g., caprylic and capric) fatty acids available under the tradename CAPMUL MCM or CAPMUL MCM90, either of which contains a minimum of 70% monoglycerides, alpha, as determined by the American Oil Chemists' Society (AOCS) Test Method Cd 11-57. CAPMUL MCM90 generally contains between 83 to 95% monoglycerides. CAPMUL MCM has a maximum acid value of 2.5.

Acid values as used in this application are determined by AOCS Test Method Cd3a-63, acid value meaning the number of milligrams of potassium hydroxide required to neutralize the free acids in 1.0 gram of the substance. See USP XXII Chemical Tests / Fats and Fixed Oils (401).

CAPMUL MCM is available from Capital City Products Co., Columbus, Ohio, or Abertech, Inc. (Karlshamn), Karshamn, Sweden. Preferably, this material has an acid value of less than or equal to about 0.5. A more preferred glyceride would be CAPMUL MCM but having an acid value of less than or equal to about 0.26, further preferably less than or equal to 0.1 and most preferably less than or equal to 0.04 Another similar material is available under the tradename IMWITTOR 988 (from Hüls, Germany) (glycerol-mono-di-caprylate), which contains a minimum of 45% of monoglycerides as determined by gas chromatography. IMWITTOR 988 has a typical composition of about 50% monoglycerides, about 40% diglycerides, and about 6% triglycerides (all percentages determined by gas chromatography). IMWITTOR 988 has a maximum acid value of about 2 (in units mg KOH/g).

The partially ethoxylated glyceride is preferably a liquid which is a mixture of mono-, di-, and triglycerides wherein the free hydroxy group is ethoxylated with ethylene glycol or ethylene oxide. More particularly, the partially ethoxylated glyceride is polyethylene glycol 300 C₈-C₁₀ medium chain fatty acid glyceride composition, the polyethylene glycol having an average molecular weight of from 300 to 500 (equivalent from 6 to 8 moles of ethylene oxide). Such is available under the trademark SOFTIGEN 767, available from Hüls AG or Hüls America, Piscataway, New Jersey. SOFTIGEN 767 is a blend of partial glycerides of natural, saturated, even numbered vegetable fatty acids with chain lengths in the C₈ to C₁₀ range (greater than 90% of C₈ and C₁₀ fatty acids and less than 2% of C₆ and C₁₂ fatty acids) and has the structure(s):

R-CH₂-CH[(O-CH₂-CH₂)pOH]-CH₂-R

R-CH₂-CH[(O-CH₂-CH₂)pOH]-CH₂-(O-CH₂-CH₂)pOH

where R is the acyl moiety of a C₈₋₁₀ fatty acid, and p is 3 or 4.

SOFTIGEN 767 is also known by its CTFA (Cosmetics, Toiletry and Fragrance Association) name PEG-6 Caprylic/Capric Glycerides. The partially ethoxylated glyceride can also be obtained under the tradename LABRASOL (saturated polyglycolyzed C₈-C₁₀ glycerides; CTFA name PEG-8 Caprylic/Capric Glycerides) available from Gattefossé Corporation, Westwood, New Jersey.

Compositions which are also contemplated by this invention include formulations, with respect to the medium chain fatty acid glycerides, of one hundred percent (100%) of mono-diglyceride of saturated C₈ to C₁₀ fatty acids (e.g, CAPMUL MCM) and zero percent (0%) of polyethylene glycol 300-caprylic/capric glyceride (e.g., SOFTIGEN 767) to one hundred percent (100%) of polyethylene glycol 300-caprylic/capric glyceride (e.g., SOFTIGEN 767) to zero percent (0%) of mono-diglyceride of saturated C₈ to C₁₀ fatty acids (e.g., CAPMUL MCM).

Compositions can also include PEG 400 and PEG 8000 (polyethylene glycols of molecular weights of about 400 and 8000), and polyoxyl(40) castor oil (which is castor oil with an average of 40-45 moles of ethylene oxide; also known as PEG-40 Hydrogenated Castor Oil (CFTA Name); available as Cremophor RH-40 from BASF). Cremophor RH-40 has the following typical physical properties: acid value of ≤ 1.0; hydroxyl value of 60-80; and saponification of 50-60. Certain other compositions can also contain polyoxyethylene-polyoxypropylene copolymer (a block polymer of ethylene oxide and propylene oxide).

Of the various unit dosage forms that one can contemplate, for example, hard gelatin capsules, soft gelatin capsules, tablets, caplets, enteric coated tablets, enteric coated hard gelatin capsules, enteric coated soft gelatin capsules, minicapsules, dragees, solutions, emulsions, suspensions, syrups, sprays, and suppositories, soft gelatin capsules, enteric coated soft gelatin capsules, minicapsules, and suppositories are preferred unit dosage forms and soft gelatin capsules and minicapsules are especially preferred unit dosage forms.

The amount of proteinase inhibitor for the unit dosage forms, with the exception of the minicapsules, range from 10 mg to 3000 mg, preferably from 25 mg to 1800 mg, more preferably from 25 mg to 600 mg, even more preferably from 50 mg to 400 mg, and even more preferably from 120 mg to 300 mg, and most preferably in an amount of 200 mg. For minicapsules, the amount of proteinase inhibitor is from 0.5 to 2 mg (which corresponds to a fill volume of 5 to 10 mg liquid). Alternatively, the size of the minicapsule can range from 0.5 mm to 5 mm in diameter, preferably from 1 to 2 mm in diameter.

For the manufacture of tablets, coated tablets, dragees and hard gelatine capsules the proteinase inhibitors can be processed with pharmaceutically inert, inorganic or organic excipients. Lactose, maize starch or derivatives thereof, talc, stearic acid or its salts etc. can be used, for example, as such excipients for tablets, dragees and hard gelatine capsules.

Suitable excipients for soft gelatine capsules are, for example, vegetable oils, waxes, fats, semi-solid and liquid polyols.

Suitable excipients for the manufacture of solutions and syrups are, for example, water, polyols, saccharose, invert sugar, and glucose.

Suitable excipients for injection solutions are, for example, water, alcohols, polyols, glycerol and vegetable oils.

Suitable excipients for suppositories are, for example, natural or hardened oils, waxes, fats, semi-liquid or liquid polyols.

Moreover, the unit dose pharmaceutical compositions can contain preserving agents, solubilizers, viscosity-increasing substances, stabilizing agents, wetting agents, emulsifying agents, sweetening agents, coloring agents, flavoring agents, salts for varying the osmotic pressure, buffers, coating agents or antioxidants. Preferably, the antioxidant is dl-alpha tocopherol which is present in the inventive formulation in an amount of from 0.01 to 0.5 % on a weight basis, preferably 0.1 % to 0.5 %. The unit dose pharmaceutical compositions can also contain still other therapeutically valuable substances.

For compositions containing from 120 mg to 300 mg of proteinase inhibitor, and preferably 200 mg, the compositions can contain the materials in the following ranges (based on weight percent of the composition):

| | |
|---|---|
| C₈-C₁₀ fatty acid monoglyceride (e.g. CAPMUL MCM; or IMWITTOR 988) | 40-80% |
| dl-α-tocopherol | 0.01-0.5% |
| Polyvinylpyrrolidone | 0-30% |
| PEG 400 | 0-30% |
| Polyoxyl(40) Castor Oil | 0-12% |
| PEG 8000 | 0-5% |
| Polyglycolyzed C₈-C₁₀ fatty acid glycerides (e.g. LABRASOL; or SOFTIGEN 767) | 0-10% |
| Polyoxyethylene-polyoxypropylene copolymer | 0-25% |

Preferably, for compositions containing about 200 mg of proteinase inhibitor, the compositions contain 40-80% C₈-C₁₀ fatty acid monoglycerides, about 0.5% dl-α-tocopherol, 0-28% PEG-400, 0-10% Polyoxyl(40) Castor Oil; and 0-30% polyvinylpyrrolidone. Additionally, preferred compositions are, with all containing about 200 mg of proteinase inhibitor, (a) about 0.5% dl-α-tocopherol and about 79.5% C₈-C₁₀ fatty acid monoglycerides; and (b) about 0.5% dl-α-tocopherol; 40-76.5% C₈-C₁₀ fatty acid monoglyercides, 0-27.5% polyoxyl(40) castor oil; and 20-30% polyvinylpyrrolidone. More preferred compositions (mg/capsule) are as follows:

**TABLE A**

| **Material** | **A23** | **A24** | **A25** |
|---|---|---|---|
| Proteinase Inhibitor | 200 | 200 | 200 |
| dl-α-tocopherol | 5 | 5 | 5 |
| CAPMUL MCM | 795 | 765 | 400 |
| PEG 400 | | | 275 |
| Polyoxyl(40) castor oil | | | 100 |
| Polyvinylpyrrolidone | | 30 | 20 |
| Total | 1000 | 1000 | 1000 |

The Examples below describe work conducted using N-tert.-butyl-decahydro-2-[2(R)-hydroxy-4-phenyl-3(S)-[[N-(2-quinolylcarbonyl)-L-asparaginyl]amino]butyl]-(4aS,8aS)-isoquinoline-3(S)-carboxamide (referred to hereinbelow as "Compound A") and its pharmaceutically acceptable salts as an example of a proteinase inhibitor.

### Example 1

The various formulations tested below and the others which are contemplated by the present invention were made in the following manner. As an example, Formulation A-63, discussed below, was made as follows:
Compound A was sieved to remove large material.

769.0 mg of CAPMUL MCM and 1.0 mg of dl-α-tocopherol, all as liquids, were placed in a suitable vessel and were heated to 55-60°C with continuous stirring. 30.0 mg of polyvinylpyrrolidone K30 (PVP K30; average molecular weight of about 30,000) was added to the vessel and stirred until dissolved. 200.0 mg of the sieved Compound A was added slowly to the liquid by careful sprinkling it into the liquid while vigorously stirring and maintaining the temperature of the liquid at 55-60°C.

When all of the Compound A had dissolved, the vessel was removed from the heat source, the stirring was stopped, and the resulting liquid was allowed to reach room temperature (about 20 °C). The cooled liquid was then filled into soft gel capsules.

A heating temperature of at least 55°C was selected to prevent lengthy dissolution time of Compound A in the glyceride.

### Example 2

Inventive formulations A-23, A-24, A-59, and A-63 are set forth in Table 1 below. Formulation A-63 was prepared as described in Example 1 above. Formulations A-23, A-24, and A-59 were prepared in a manner analogous to that described in Example 1.

**TABLE 1**

| MATERIAL | mg/capsule | | | |
|---|---|---|---|---|
| | Formulation A-23 | Formulation A-24 | Formulation A-59 | Formulation A-63 |
| Compound A | 200.0 | 200.0 | 200.0 | 200.0 |
| CAPMUL MCM90 | 795.0 | 765.0 | 599.2 | 769.0 |
| DL-α-tocopherol | 5.0 | 5.0 | 0.8 | 1.0 |
| PVP K30 | | 30.0 | | 30.0 |
| TOTAL | 1000.0 | 1000.0 | 800.0 | 1000.0 |

### Example 3

A human volunteer study was carried out using inventive formulation A-63 as compared against noninventive formulation Formulation A-14 (Compound A-mesylate salt, 235.337 mg [equivalent to 200.000 mg of Compound A] in a carrier of anhydrous lactose, 63.300 mg; microcrystalline cellulose (Avicel PH 102), 60.000 mg; sodium starch glycolate, 16.000 mg; Povidone (polyvinylpyrrolidone) K30, 8.000 mg; and magnesium stearate, 4.000 mg; (purified water used for processing, 92.590 mg) placed in a hard gelatin capsule) to assess bioavailability.

Twelve male volunteers were used in each study, each given the equivalent of 600 mg Compound A after food: that is three capsules each of the 200 mg hard gelatin capsule Formulation A-14 and three capsules each of the 200 mg soft gelatin capsules of Formulation A-63. The 200 mg indicating that each capsule, hard or soft, contain the equivalent of 200 mg of Compound A.

After a fourteen day screening period, each subject received a single dose of either inventive formulation Formulation A-63 or non-inventive formulation Formulation A-14 within five minutes of a standardized meal (either (1) breakfast:: bowl of corn flakes with 100 ml of whole milk; two rashers of bacon; two fried eggs; two slices of toast with butter; 100 ml orange juice; and 150 ml of decaffeinated coffee or tea or (2) lunch: 200 ml of soup; sandwiches made from four pieces of bread; a chocolate biscuit; an orange or apple; and 150 ml decaffeinated coffee or tea).

Blood samples were taken immediately before dosing and at 0.5, 1, 1.5, 2, 2.5, 3, 3.5, 4, 4.5, 5, 6, 7, 8, 10, 12, 16, 20, and 24 hours after the dose. After a minimum six day washout period, each volunteer received a second dose of each relevant formulation after a standardized meal and blood sampling was done at the times previously mentioned. After another minimum six day washout period, each volunteer received a second dose of each relevant formulation after a standardized meal and blood sampling was done at the times previously mentioned.

The plasma samples of Compound A in biological fluid were analyzed by solid phase sample preparation and high performance liquid chromatography with ultraviolet detector at 238 nm (HPLC/UV) as described below.

C₈ Advanced Automated Sample Processor (AASP) cartridges were used to extract Compound A from plasma samples. The limits of quantification were 0.5 ng/ml with 1.0 ml of plasma with a precision and accuracy of 0.5% and 99.1%, respectively.

Venous blood samples (10 ml) were taken into either glass Becton Dickinson Vacutainers or polypropylene Sarstadt monovettes containing lithium heparin as an anticoagulant, and put on ice. The samples were centrifuged within 1 hour of collection at 1500 g and the plasma transferred to 5 ml polystyrene screw top tubes and stored frozen at -20°C. Prior to transfer to the analytical laboratory, plasma samples from HIV positive patients were inactivated by heat treatment in a water bath for 2 hours at 56°C (± 1°C) to destroy any HIV present. The samples were then refrozen at -20°C. Prior to analysis at the analytical laboratory, the samples were subjected to the same heat treatment.

A 300 µl aliquot of 0.5 molar monochloroacetic acid was added to each 1 ml aliquot of plasma, standard, or unknown and the samples were vortexed and then centrifuged for 3 minutes in a microfuge (MSE).

Using a AASP prep station (Varian, Walton on Thames, England), C₈ bonded phase AASP cassettes (Jones Chromatography, Hengoed, Wales) were primed with 2 x 1 ml of methanol and 2.x 1 ml of 0.001 M ammonium acetate (pH 3) buffer. The plasma samples were then loaded onto the cassette and washed with 2 x 1 ml of 0.01 M ammonium acetate and 1 ml of 0.01 M ammonium acetate (pH 3) buffer:methanol (60:40). The cassettes were transferred to the AASP system and the samples eluted into the HPLC system (Kratos Spectroflow 400 pump, ABI, Warrington, England; AASP mininjector, Varian, Walton on Thames, England; LKB 2141 variable wavelength detector, Pharmacia/LKB, Milton Keynes, England).

The AASP cassettes were purged with aliquots of methanol:water:glacial acetic acid (48.8:48.8:2.4) set for 10 cycles pre-injection and 10 cycles post-injection. The valve reset time was sent to 1 minute with a typical cycle time and run time of 15 minutes.

The eluent was methanol:0.01 M ammonium acetate:glacial acetic acid (90:9.75:0.25), at a flow rate of 2 ml/min, which resulted in a back pressure of approximately 800 psi. A Phenyl Nova-Pak Guard-Pak (Waters, Harrow, England) was used with a Phenyl Nova-Pak cartridge (4 micron) (Waters, Harrow, England) fitted with in a Waters RCM 8 x 10 compression unit (Waters, Harrow, England). The UV detector was set at 238 nm. These conditions resulted in a retention time of about 10 minutes for Compound A. The retention time was dependent upon the analytical column (due to the number of residual silanol groups), the concentration of ammonium acetate in the mobile phase, and the amount of methanol. The optimum separation of Compound A from endogenous components was achieved with a retention time of 10 minutes, accomplished by varying the concentration of ammonium acetate used to prepare the mobile phase. The concentration of ammonium acetate was established for each analytical column and was between about 0.006 and about 0.019 M.

The peak heights of Compound A were calculated automatically by computing integrator (Maxima 820 chromatography data acquisition system, Waters, Harrow, England). Peak heights or peak height ratios versus concentrations of Compound A were used to construct linear calibration curves and calculation of subsequent patient sample concentrations using RODAS suite of programs (Roche Products Ltd., Welwyn Garden City, England). Calibration curves were established using an iteratively reweighed linear least squares regression. The time for maximum concentration (Cₘₐₓ), the time to achieve maximum plasma concentration Tₘₐₓ, and area under the plasma concentration - time profile (AUC) was determined from plasma concentration - time profile of each formulation. Statistical analyses, e.g., mean, % coefficient of variability and median were also calculated.

Relevant pharmacokinetic parameters of inventive formulation Formulation A-63 as compared to noninventive formulation Formulation A-14 are found in Table 2.

**TABLE 2**

| Formulation | Statistic | Parameter (units) | | | |
|---|---|---|---|---|---|
| | | Cₘₐₓ | Tₘₐₓ | tlag | AUC* |
| | | (ng/mL) | (h) | (h) | (ng.h/mL) |
| Formulation A-63 | Mean | 334.6 | -- | -- | 701.4 |
| | %CV | 70.9 | -- | -- | 80.9 |
| | Median | 273.6 | 1.5 | 0 | 530.1 |
| Formulation A-14 | Mean | 61.85 | -- | -- | 194.9 |
| | %CV | 62.3 | -- | -- | 59.3 |
| | Median | 57.17 | 4.5 | 0.5 | 189.9 |

| | | | | | |
|---|---|---|---|---|---|
| *AUC calculated to the last measurable plasma concentration. | | | | | |

The data of Table 2 show inventive formulation Formulation A-63 shows a Cₘₐₓ over five times that of noninventive Formulation A-14. Inventive formulation Formulation A-63 shows a AUC over 3.5 times that of noninventive formulation Formulation A-14. The results of this evaluation were surprising.

The Compound A-mesylate salt (used in non-inventive Formulation A-14) has a greater solubility than Compound A base (2.55 vs. 0.03 mg/ml at room temperature in water; 0.06 vs. 0.05 mg/ml at room temperature in pH 1.1 (HCl); and 2.32 vs < 0.01 in pH 5.4 phosphate buffer). The mesylate salt of Compound A was used in Formulation A-14 due to its greater solubility in aqueous medium compared to Compound A base. If the Compound A base in non-inventive Formulation A-14 would have been used, the bioavailability would have been less than shown in Table 2 because of its low aqueous solubility. However, in inventive formulation Formulation A-63 which contains glycerides as a vehicle, Compound A as a free base has greater solubility than the mesylate salt of Compound A.

### Example 4

Other formulations contemplated by the present invention are as follows:

**TABLE 3**

| Item # | MATERIAL (mg/capsule) | Formulation A-25 | Formulation A-28 | Formulation A-29 |
|---|---|---|---|---|
| 1 | Compound A | 200.00 | 200.00 | 200.00 |
| 2 | CAPMUL MCM | 400.00 | 750.00 | 550.00 |
| 3 | PEG 400 | 275.0 | 0 | 0 |
| 4 | PEG 8000 | 0 | 49.65 | 0 |
| 5 | PEG (40) Castor Oil | 100.0 | 0 | 0 |
| 6 | Polyoxyethylene-Polyoxypropylene Copolymer | 0 | 0 | 249.65 |
| 7 | PVP 30 | 20.0 | 0 | 0 |
| 8 | dl-α-Tocopherol | 5.0 | 0.35 | 0.35 |
| | Total Fill Weight (mg) | 1000.0 | 1000.0 | 1000.0 |

The formulations of Table 3 can be made in the following manner:
Mix Items 2, 3, 4, 5, 6 and 7 in a suitable vessel with stirring and warm the mixture to about 50°C. Add Compound A (item 1) to the mixture while mixing until clear solution is obtained. Then cool the mixture to room temperature. Then add dl-a-tocopherol (item 8) and mix until dissolved. Fill in size 16 oblong soft gelatin capsule.

### Example 5

Additional examples of other formulations contemplated by the present invention are as follows:

**TABLE 4**

| Ingredient | mg/capsule | | | |
|---|---|---|---|---|
| Compound A | 200 | 200 | 200 | 300 |
| CAPMUL MCM | 795 | 400 | 400 | 695 |
| PEG(40) Castor Oil | 0 | 120 | 0 | 0 |
| PEG 400 | 0 | 275 | 295 | 0 |
| PEG (8) caprylic/caproic glyceride | 0 | 0 | 100 | 0 |
| dl-α-tocopherol | 5 | 5 | 5 | 5 |
| TOTAL | 1,000 | 1,000 | 1,000 | 1,000 |

The formulations of Table 4 can be made in the following manner:
Mix CAPMUL MCM, and where used, PEG(40) Castor Oil, PEG 400, PEG (8) caprylic/caproic glyceride, and dl-α-tocopherol in a suitable vessel with stirring and warm the mixture to about 50°C. Add Compound A to the mixture while mixing until clear solution is obtained. Then cool the mixture to room temperature. Fill in appropriately sized hard or soft gelatin capsules. The hard gelatin capsules can be sealed to prevent any leakage of the fill material.

While a number of embodiments of this invention are described herein, it is apparent that the embodiments can be altered to provide other embodiments that utilize the compositions and processes of this invention.

## Claims

1. A unit dose pharmaceutical composition comprising:
(a) a therapeutically effective amount of N-tert.-butyl-decahydro-2-[2(R)-hydroxy-4-phenyl-3(S)- [[N-(2-quinolylcarbonyl)-L-asparaginyl]amino]butyl]-(4aS,8aS)-isoquinoline-3(S)-carboxamide its pharmaceutically acceptable salts or esters (including their salts) or ofN-tert.-butyl-decahydro-2-[2(R)-hydroxy-4-phenyl-3(S- [[N-benzyloxycarbonyl)-L-asparaginyl]-amino]butyl]-(4aS,8aS)-isoquinoline-3(S)-carboxamide, its pharmaceutically salts or esters (including their salts)]; and
(b) a pharmaceutically acceptable carrier having a monoglyceride of a C₈ - C₁₀ medium chain fatty acid, wherein the monoglyceride is present in an amount which is sufficient to dissolve the proteinase inhibitor.

2. A composition of claim 1 wherein the ratio of the monoglyceride (b) to (a) is at least 1.5 by weight.

3. A composition of claim 2 wherein the ratio of the monoglyceride of (b) to (a) is at least 3.0

4. A composition of any one of claims 1-3 wherein the acid value of (b) is less than or equal to 0.04.

5. A composition of any one of claims 1-4 which further comprises polyvinylpyrrolidone.

6. A composition of any one of claims 1-5 further comprising 0.01 to 0.5 % dl-α-tocopherol by weight of the composition.

## Patentansprüche

1. Eine pharmazeutische Zusammensetzung als Dosierungseinheit, enthaltend:
(a) ein therapeutisch wirksame Menge an N-tert.-Butyl-decahydro-2-[2(R)-hydroxy-4-phenyl-3(S)-[[N-(2-chinolylcabonyl)-L-asparaginyl]amino]butyl]-(4aS,8aS)-isochinolin-3(S)-carboxamid, dessen pharmazeutisch akzeptablen Salzen oder Estern (einschließlich deren Salze) oder an N-tert.-Butyldecahydro-2-[2(R)-hydroxy-4-phenyl-3(S-[[N-benzyloxycarbonyl)-Lasparaginyl]-amino]butyl]-(4aS,8aS)-isochinolin-3(S)-carboxamid, dessen pharmazeutischen Salzen oder Estern (einschließlich deren Salze)]; und
(b) einen pharmazeutisch akzeptablen Träger, der ein Monoglycerid einer Fettsäure mittlerer Kettenlänge von C₈-C₁₀ aufweist, wobei das Monoglycerid in einer Menge vorliegt, die zum Lösen des Proteinase-Inhibitors ausreichend ist.

2. Eine Zusammensetzung nach Anspruch 1, wobei das Gewichts-Verhältnis von dem Monoglycerid (b) zu (a) wenigstens 1,5 beträgt.

3. Eine Zusammensetzung nach Anspruch 2, wobei das Verhältnis von dem Monoglyderid gemäß (b) zu (a) wenigstens 3,0 beträgt.

4. Eine Zusammensetzung nach irgendeinem der Ansprüche 1 - 3, wobei der Säurewert von (b) kleiner als oder gleich 0,04 ist.

5. Eine Zusammensetzung nach irgendeinem der Ansprüche 1 - 4, die ferner Polyvinylpyrrolidon enthält.

6. Eine Zusammensetzung nach irgendeinem der Ansprüche 1 - 5, die ferner 0,01 bis 0,5% des Gewichts der Zusammensetzung an dl-α-Tocopherol enthält.

## Revendications

1. Composition Pharmaceutique en dose unitaire comprenant :
(a) une quantité thérapeutiquement efficace du N-tert-butyl-décahydro-2-[2(R)-hydroxy-4-phényl-3(S)-[[N-(2-quinoléylcarbonyl)-L-asparaginyl]amino]butyl]-(4aS,8aS)-isoquinoléine-3(S)carboxamide, de ses sels ou de ses esters (y compris leurs sels) pharmaceutiquement acceptables ou du N-tert-butyl-décahydro-2-[2(R)-hydroxy-4-phényl-3(S)-[[N-benzyloxycarbonyl-L-asparaginyl]amino]butyl]-(4aS,8aS)-isoquinoléine-3(S)carboxamide, de ses sels ou de ses esters (y compris leurs sels) pharmaceutiquement acceptables ; et
(b) un support pharmaceutiquement acceptable comprenant un monoglycéride d'un acide gras à chaîne moyenne en C₈-C₁₀, le monoglycéride étant présent en une quantité suffisante pour dissoudre l'inhibiteur de protéinase.

2. Composition selon la revendication 1, dans laquelle le rapport du monoglycéride (b) à (a) est d'au moins 1,5 en poids.

3. Composition selon la revendication 2, dans laquelle le rapport du monoglycéride (b) à (a) est d'au moins 3,0.

4. Composition selon l'une quelconque des revendications 1 à 3, dans laquelle l'indice d'acide de (b) est égal ou inférieur à 0,04.

5. Composition selon l'une quelconque des revendications 1 à 4, qui comprend en outre de la polyvinylpyrrolidone.

6. Composition selon l'une quelconque des revendications 1 à 5, comprenant en outre 0,01 à 0,5 % en poids de dl-α-tocophérol par rapport à la composition.
